# EUROPEAN PATENT APPLICATION

(11) **EP 1 284 284 A2**
(43) Date of publication of application: **19.02.2003**
(21) Application number: 02018483.4
(22) Date of filing: 16.08.2002
(51) Int. Cl.: C12N 5/06, C12Q 1/02

(54) **Cell culture model for arteriosclerosis**

(30) Priority: 17.08.2001 JP 2001248046
(71) Applicant: Fuji Photo Film Co. Ltd., Kanagawa 250-0193 (JP)
(72) Inventor: Aikawa, Kazuhiro, Minami-ashigara-shi, Kanagawa 250-0193 (JP); Kitaguchi, Hiroshi, Minami-ashigara-shi, Kanagawa 250-0193 (JP)
(74) Representative: HOFFMANN - EITLE

(57) **Abstract**

Disclosed is a method for preparing a cell culture system, which comprises the step of simultaneously culturing two or more kinds of cell species (e.g., macrophages and vascular smooth muscle cells) which are involved in formation of a lesion of a mammalian disease (e.g., formation of an arteriosclerotic lesion) in one cell culture vessel under separation of said two or more kinds of cells by using, for example, a cell filter. A cell culture system is provided in which an environment of a lesion of a particular disease is reproduced.

## Description

### Field of the Invention

The present invention relates to a cell culture system. More specifically, the present invention relates to a cell culture system reproducing an environment of a lesion of a particular disease, which is useful for evaluating effectiveness and distribution of a medicament into a lesion.

### Related Art

In the modern society, especially in the societies of advanced countries, opportunities of ingesting high calorie and high fat diet are increasing. For this reason, mortalities due to ischemic diseases resulting from arteriosclerosis (heart diseases such as myocardial infarction and angina pectoris, cerebrovascular diseases such as cerebral infarction and cerebral hemorrhage) have been increasing. Therefore, it has been desired to diagnose such conditions at an early stage to employ an appropriate treatment. However, no satisfactory method is available for diagnosing progress of arteriosclerosis at an early stage before the onsets of the aforementioned diseases.

Methods for diagnosing arteriosclerosis are basically classified into non-invasive methods and invasive methods in which a catheter or the like is inserted into an artery. Among them, typical non-invasive methods include X-ray angiography and ultrasonography. However, by these methods, it is almost impossible to detect arteriosclerosis at an early stage, especially constriction of coronary artery, that causes myocardial infarction or angina pectoris, at an early stage before the onset of these diseases.

CT, MRI and the like may sometimes be used as another class of non-invasive methods. However, these methods have been mainly developed for detection of tumors, and accordingly, they have a problem of a low resolution of arteriosclerotic lesions. In addition, the methods require expensive and large-scale apparatuses, which limits employable hospitals and general applicability. In addition, methods utilizing radioisotopes have also been investigated, however, they still remain at an experimental level.

As the invasive methods, intravascular echo, vascular endoscope and the like have been used. It is recognized that an arteriosclerotic lesion with a thickness as thin as 0.1 mm can be measured by these methods. However, for employment of these methods, it is necessary to arterially insert an ultrasonic oscillator or an endoscope attached to an end of a catheter, which may result in serious physical stress and heaviness as well as a risk of a patient. Therefore, although these methods have been used therapeutically for patients after the attack of myocardial infarction and the like or as secondary prophylaxis, they cannot be used for a diagnostic purpose to know as to presence or absence or a degree of progress of arteriosclerosis in a patient before onset.

Among the aforementioned methods, a method most widely used for identification of a lesion of arterial vasoconstriction is X-ray angiography. This method comprises the step of administration of a water-soluble iodine contrast medium to visualize vascular flows, and detecting a lesion at which the flows are obstructed. However, generally, this method can only detect a lesion where constriction progresses 50% or more and fails to detect a lesion before the onset of attack of an ischemic disease.

Separately from these methods, some attempts have also been reported in which a hydrophobic iodine contrast medium or a hydrophilic contrast medium is formulated for selective accumulation in a target pathological lesion (International Patent Publications WO95/19186, WO95/21631, WO89/00812 British Patent No. 867650, WO96/00089, WO94/19025, WO96/40615, WO95/2295, WO98/41239, WO98/23297, WO99/02193, WO97/06132, U.S. Patent Nos. 4,192,859, 4,567,034, 4,925,649, Pharm. Res., 16 (3), 420 (1999), J. Pharm. Sci., 72 (8), 898 (1983), Invest. Radiol., 18 (3), 275 (1983)). For example, Pharm. Res., 16 (3), 420 (1999) discloses that by injection of an oil-particle dispersion of cholesteryl iopanoate as a hydrophobic compound, the iodine compound accumulates in arteriosclerotic lesions of experimental animals.

Further, J. Pharm. Sci. 72 (8), 898 (1983) discloses examples of X-ray hepatography and splenography by injection of an oil-particle dispersion of cholesteryl iopanoate. U.S. Patent No. 4,567,034 describes a method of selective hepatography or splenography utilizing liposomes encapsulating an ester of diatrizoic acid. International Patent Publications WO96/28414 and WO96/00089 disclose contrast media for imaging vascular pools or lymphatic systems. However, the methods using these formulations are not satisfactory in efficiency and selectivity for a purpose of selective contrast of vascular diseases, and no example thereof is reported in which vascular diseases are imaged by utilizing X-ray irradiation.

Mechanisms of onset of arterial diseases have recently been being elucidated at each levels of genes, proteins, and cells (J. Biol. Chem., 1996, 271 (44) 27346-52; Nature, 386 (6662) 292-6). As for arteriosclerosis, it has been elucidated that plural kinds of cells form lesions under mutual control of proliferation (Arterioscler. Throm. Vasc. Biol., 1999 (3) 461-71; Lab Invest 1998, 78 (4) 423-34). No reproduction of environments of a lesion in a cell culture vessel, in which plural kinds of cells are involved, has been reported, and evaluation of medicaments for arteriosclerosis or restenosis have so far been conducted mainly by using model animals.

However, any methods using animals are time-consuming and expensive, and are required to be essentially minimized from a viewpoint of prevention of cruelty of animals. Therefore, an in vitro evaluation method, in which environments of arteriosclerotic lesion are reproduced, has been desired for screening numbers of compounds for a short period of time.

### Summary of the Invention

An object of the present invention is to provide a cell culture system reproducing an environment of a lesion of arteriosclerosis, restenosis or the like for which formation plural kinds of cells are involved. Another object of the present invention is to provide a method for evaluating a medicament for animal diseases by applying the aforementioned means. The inventors of the present invention conducted various researches to achieve the foregoing objects, and as a result, they achieved the present invention described below.

The present invention thus provides a method for preparing a cell culture system which comprises a step of simultaneously culturing, in a single cell culture vessel, two or more kinds of cell species which are involved in formation of a lesion of a mammalian disease. As preferred embodiments of the present invention, provided are the aforementioned method wherein the cell species are primary culture cells isolated from a living body or established subculture cells; the aforementioned method wherein the cell species are mammalian primary culture cells; and the aforementioned method wherein the mammalian disease is a human disease.

According to more preferred embodiments of the present invention, provided are the aforementioned method wherein at least one of the cell species is a cell which is involved in formation of arteriosclerotic lesion; the aforementioned method wherein the cell species are selected from the group consisting of a vascular endothelial cell, a vascular smooth muscle cell, an adipocyte cell, a hemocyte cell, and a macrophage; the aforementioned method wherein the cell species are selected from the group consisting of a vascular endothelial cell, a vascular smooth muscle cell, and a macrophage; the aforementioned method wherein two kinds of cell species are cultured in a single culture vessel under separation of said two kinds of cells by using a cell filter; and the aforementioned method wherein the two kinds of cell species are a macrophage and a vascular smooth muscle cell. According to a particularly preferred embodiment of the present invention, provided is a method for preparing a cell culture system in which an environment of an arteriosclerotic lesion is reproduced, which comprises the step of culturing, in a single culture vessel, two kinds of mammalian primary culture cells which are involved in formation of an arteriosclerotic lesion under separation of said cells by using a cell filter.

The present invention further provides a cell culture system which is obtainable by the aforementioned method for preparation of a cell culture system. The present invention also provides a method for screening a medicament by utilizing the cell culture system that is obtainable by the aforementioned method for preparation of a cell culture system. According to preferred embodiments, the present invention provides a method for judgment of effectiveness of a medicament on a vascular disease, which comprises the step of determining an action of the medicament on vascular smooth muscle cells which are proliferated by culturing a macrophage, preferably a foamed macrophage, and the vascular smooth muscle cell under separation of said two kinds of cells by using a cell filter; and a method for evaluation of distribution of a medicament into a lesion of a vascular disease, which comprises the step of determining an action of the medicament on vascular smooth muscle cells which are proliferated by culturing a macrophages, preferably a foamed macrophage, and the vascular smooth muscle cell under separation of said two kinds of cells by using a cell filter.

The cell culture system provided by the method for preparation of a cell culture system of the present invention can be utilized for in vitro screening of a medicament or the like as a cell culture system in which an environment of a lesion of arteriosclerosis, restenosis or the like is reproduced.

### Brief Explanation of Drawings

Fig. 1 shows results of induction of mouse vascular smooth muscle cell proliferation in the presence of mouse foamed macrophages.

Fig. 2 shows a proliferation curve of mouse vascular smooth muscle cells without addition of mouse formed macrophages.

Fig. 3 shows results of induction of rat vascular smooth muscle cell proliferation in the presence of rat foamed macrophages.

Fig. 4 shows results of induction of rabbit vascular smooth muscle cell proliferation in the presence of rabbit foamed macrophages.

### Preferred Embodiments of the Invention

The method for preparing a cell culture system provided by the present invention is characterized to comprise a step of culturing, in a single cell culture vessel, two or more kinds of cell species which are involved in the formation of a lesion of a mammalian disease. As the cell species, a primary culture cell and an established subculture cell are preferred, and a mammalian primary culture cell is particularly preferred. At least one of the two or more kinds of cell species may preferably be a primary culture cell. Origins of the cell species are not particularly limited, and preferred mammals include human, dog, cat, pig, miniature pig, rabbit, hamster, rat, mouse and the like. However, mammals are not limited to these examples.

The two or more kinds of distinguishable cell species to be cultured in a single culture vessel may be derived from homologous animals or heterogenous animals. Those derived from homologous animals are preferred. In the specification, the term "involved in formation of a lesion" should be construed in its broadest sense, including such as where plural kinds of cells form a lesion under mutual control of proliferation, and the term should not be construed in any limitative sense. As the distinguishable cell species, cells distinguishable from a viewpoint of cellular biology or cytotaxonomy may preferably be chosen. More preferably, two kinds of cells may be chosen which coexist in a particular lesion and will form the lesion under mutual control of proliferation.

According to a preferred embodiment of the present invention, two kinds of cell species are used, and preferably they are involved in the formation of an arteriosclerotic lesion. Preferred cell species include vascular endothelial cells, vascular smooth muscle cells, adipocyte cells, hemocyte cells, macrophages, or other cells that constitute tissues of blood vessels. For example, macrophages and vascular smooth muscle cells, which coexist in an arteriosclerotic lesion, may preferably be chosen as the two kinds of cell species. Either of foamed macrophages or non-foamed macrophages may be used. Foamed macrophages are preferred. Further, origins of the macrophages are not particularly limited, and any of those derived from abdominal cavity, blood, lymph node and the like may be used. Macrophages derived from abdominal cavity are preferred. Vascular smooth muscle cells may be primary culture cells or established subculture cells. Primary culture cells are preferred.

According to a preferred embodiment of the method for preparing a cell culture system of the present invention, two kinds of cell species can be cultured in a single culture vessel under separation of each cell species with a cell filter. Types of the cell filters are not particularly limited so long as the filters have such a pore size that prevents passage of foamed macrophages and vascular smooth muscle cells. An example of the pore size that prevents passage of the cells includes a range of 0.2-10 µ m. Depending on types of the cells to be used, such as formed macrophages, a cell filter having an appropriate pore size can be readily chosen.

Culture vessels used for the present invention are not particularly limited. For example, a culture flask, a culture tube, a dish, a microplate and the like are preferred, and among them, a dish and a microplate are particularly preferred. Materials of the culture vessels are not particularly limited. For example, hard glass, polystyrene, polypropylene, polyethylene, and polyvinyl chloride plastic are preferred, and among them, polystyrene and polypropylene are particularly preferred. Types of mediums used are not particularly limited. For example, Eagle's MEM, Dulbecco's modified Eagle's MEM, RPMI1640, F-10, F-12 McCoy's medium, NCTC-109, William's medium and the like are preferred, and Eagle's MEM, Dulbecco's modified Eagle's MEM and the like are particularly preferred. When serum is used, calf serum, FBS, mouse serum, rat serum, rabbit serum, human serum, horse serum and the like, for example, are preferred, and among them, calf serum and FBS are particularly preferred. As a combination where macrophages and vascular smooth muscle cells are used, for example, a combination of a microplate made of polystyrene, Eagle's MEM, and FBS is preferred.

Cell numbers of the two kinds of cell species are not particularly limited. When macrophages and vascular smooth muscle cells are used, for example, the numbers of cells to be inoculated are preferably in the range of 0.5 to 7 × 10⁵ cells/well for macrophages and 0.5 to 8 × 10⁴ cells/well for vascular smooth muscle cells, more preferably, in the range of 1 to 4 × 10⁵ cells/well for macrophages and 0.8 to 4 × 10⁴ cells/well for vascular smooth muscle cells. Each of the macrophages and vascular smooth muscle cells may be inoculated on either of an upper or lower layer of a cell filter. Macrophages are preferably inoculated on an upper layer of a cell filter and vascular smooth muscle cells are preferably inoculated on a lower layer of the cell filter. A time of the inoculation of each of the cell species is not particularly limited. Preferably, vascular smooth muscle cells are antecedently inoculated. For example, vascular smooth muscle cells are preferably inoculated 1 to 4 days, more preferably 2 or 3 days before the inoculation of macrophages. A method is most preferred which comprises the steps of inoculating 1 to 2 × 10⁴ cells/well of vascular smooth muscle cells on a lower layer of a cell filter, culturing said cells for 3 days, and then inoculating 1.8 to 2.2 × 10⁵ cells/well of macrophages on an upper layer to prepare a cell culture system.

According to the present invention, a method for evaluating distribution of a medicament into a lesion of a vascular disease, which comprises the step of measuring an action of the medicament on vascular smooth muscle cells which are proliferated by culturing foamed macrophages and the vascular smooth muscle cells under separation of said two kinds of cells by using a cell filter. The term "action of a medicament" used in the specification should be construed in its broadest sense including therapeutic effect, diagnostic effect and the like.

Although it is not intended to be bound by any specific theory, when macrophages and vascular smooth muscle cells are cultured under separation by using a cell filter, a proliferation-activating substance derived from the foamed macrophages acts on the vascular smooth muscle cells to induce proliferation, and as a result, abnormal proliferations of vascular smooth muscle cells, which are occurred in a vascular disease such as arteriosclerosis and restenosis after PTCA, can be reproduced in vitro. Investigation of uptake of a medicament by the macrophages or the proliferated vascular smooth muscle cells enables screening of a medicament having high effectiveness on vascular diseases such as arteriosclerosis and restenosis after PTCA.

### EXAMPLES

The present invention will be explained more specifically with reference to the following examples. However, the scope of the present invention is not limited to the following examples.

### Example 1: Preparation of a culture system of vascular smooth muscle cells of which proliferation is activated by foamed macrophages

Vascular smooth muscle cells were isolated from mouse aorta endothelium ("Tissue Culture Method", 10th Edition, ed. by the Japanese Tissue Culture Association, published by Kodansha, 1998). The isolated vascular smooth muscle cells were suspended in 10% FBS Eagle's MEM (GIBCO, No. 11095-080) and inoculated in each well of a 12-well microplate (FALCON, No. 3503). The number of the cells in each well was adjusted to 10,000 cells. The cells were cultured for 3 days under conditions of 37°C and 5% CO₂.

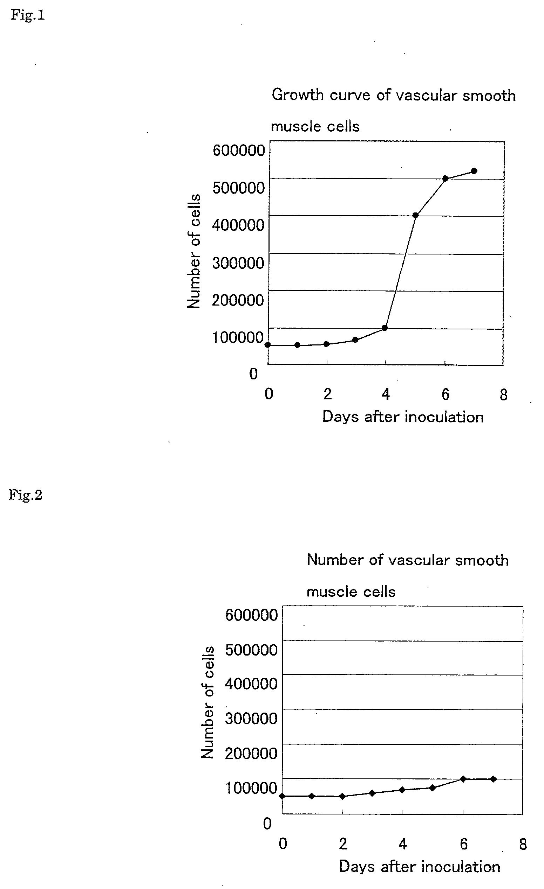

Then, foamed mouse peritoneal macrophages were prepared according to the method described in Biochimica Biophysica Acta, 1213, 127 (1994). 200,000 cells of the foamed macrophages were separated, and then inoculated on an insert cell (FALCON, No. 3180) placed at upper position of each well of the microplate in which the vascular smooth muscle cells were cultured on the bottom surface. The cells were cultured for 5 days under conditions of 37°C and 5% CO₂. The cell numbers of the vascular smooth muscle cells in the above experiment are shown in Fig. 1. Although the cells proliferated gently at the beginning of the culture, they actively proliferated after the addition of the foamed macrophages on the 3rd day with an induction period of one day. A proliferation curve of the vascular smooth muscle cells without the addition of the macrophages is shown in Fig. 2. Comparison of the results shown in Figs. 1 and 2 clearly indicates an activating effect on proliferation by the foamed macrophages.

### Example 2: Observation of expression of scavenger receptors on vascular smooth muscle cells

It is known that vascular smooth muscle cells in an arteriosclerotic lesion express scavenger receptors on their surfaces to uptake oxidized LDL (Biochem. Phamacol., 15:57 (4), 383 (1999); Exp. Mol. Pathol., 64 (3), 127, 1997). The vascular smooth muscle cells in the culture system of Fig. 1 were immunostained by using mouse scavenger receptor antibodies. As a result, although the expression was not observed in the vascular smooth muscle cells on the 3rd day from the inoculation, clear staining was observed on the 6th day from the inoculation. When the foamed macrophages on the cell filter were immunostained in the same manner, clear staining was also observed.

### Example 3: Uptake of LDL by vascular smooth muscle cells

In the culture system of Fig. 1, ¹²⁵I-labeled oxidized LDL was added to the medium for the vascular smooth muscle cells on the 3rd day and 6th day from the inoculation. ¹²⁵I taken up into the cells was counted 24 hours after each addition. The results are shown in Table 1. Clear difference in the uptake amount was observed between the results on the 3rd day and 6th day. These results indicate that the vascular smooth muscle cells cultured in the cell culture system of the present invention had properties similar to those of smooth muscle cells in a lesion of arteriosclerosis, restenosis or the like.

**Table 1**

| | Uptake of ¹²⁵I-oxLDL (× 1,000 cpm) |
|---|---|
| 3 Days after inoculation | 0.52±0.11 |
| 6 Days after inoculation | 2.2 ±0.4 |

### Example 4: Preparation of cell culture system of vascular smooth muscle cells of which proliferation is activated by foamed macrophages (2)

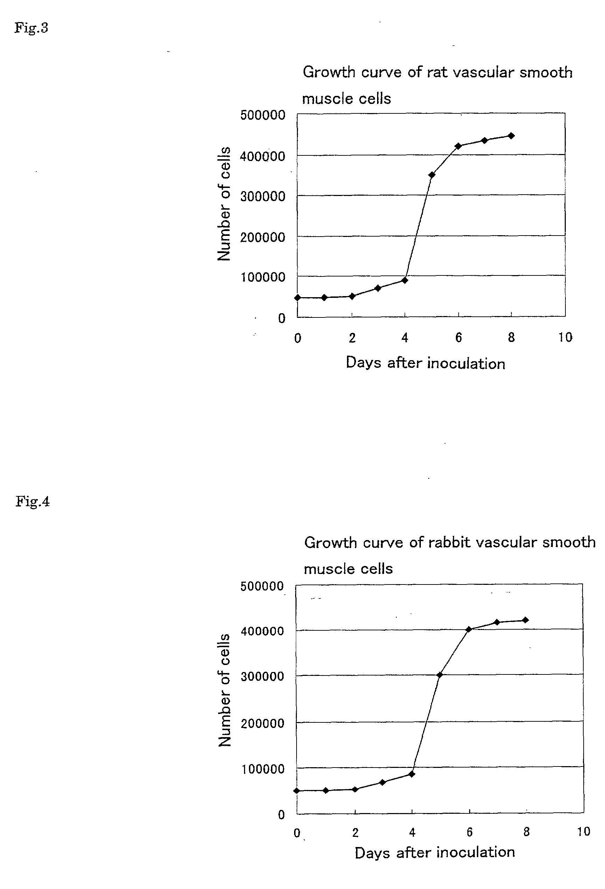

In the same manner as in Example 1, culture systems comprising rat or rabbit vascular smooth muscle cells and macrophages were prepared. The cell numbers of the vascular smooth muscle cells are shown in Figs. 3 and 4. Each of the experiments gave similar results to those obtained for the mouse shown in Fig. 1. The vascular smooth muscle cells cultured in the culture system of Fig. 1 had properties of healthy blood vessel until the 3rd day from the inoculation, and properties of smooth muscle cells in an arteriosclerotic lesion after the 7th day from the inoculation. Accordingly, experiments for screening a medicament effective selectively on the lesion can be performed by comparing actions on each of the cells.

## Claims

1. A method for preparing a cell culture system, which comprises the step of simultaneously culturing, in a single cell culture vessel, two or more kinds of cell species which are involved in formation of a lesion of a mammalian disease.

2. The method according to claim 1, wherein the cell species are primary culture cells isolated from a living body.

3. The method according to claim 1 or 2, wherein at least one of the cell species is a cell which is involved in formation of an arteriosclerotic lesion.

4. The method according to any one of claims 1 to 3, wherein the cell species are selected from the group consisting of a vascular endothelial cell, a vascular smooth muscle cell, an adipocyte cell, a hemocyte cell, and a macrophage.

5. The method according to any one of claims 1 to 4, wherein two kinds of the cell species are cultured in a single culture vessel under separation of said two kinds of cells by using a cell filter.

6. The method according to claim 5, wherein the two kinds of the cell species are a macrophage and a vascular smooth muscle cell.

7. A method for preparing a cell culture system in which an environment of an arteriosclerotic lesion is reproduced, which comprises the step of culturing two kinds of mammalian primary culture cells which are involved in formation of an arteriosclerotic lesion in a single culture vessel under separation of said two kinds of cells by using a cell filter.

8. A cell culture system which is obtainable by the method according to claim 7.

9. A method for screening a medicament by using the cell culture system according to claim 8.

10. A method for judgment of effectiveness of a medicament on a vascular disease, which comprises the step of determining an action of the medicament on a vascular smooth muscle cell which is proliferated by culturing a macrophage and the vascular smooth muscle cell under separation of said two kinds of cells by using a cell filter.

11. A method for evaluation of distribution of a medicament into a lesion of a vascular disease, which comprises the step of determining an action of the medicament on a vascular smooth muscle cell which is proliferated by culturing a macrophage and the vascular smooth muscle cell under separation of said two kinds of cells by using a cell filter.
